Europäisches Patentamt

European Patent Office

Office européen des brevets

(11) Veröffentlichungsnummer : **0 047 834**
**B1**

(12) # EUROPÄISCHE PATENTSCHRIFT

(45) Veröffentlichungstag der Patentschrift :
20.02.85

(51) Int. Cl.⁴ : **C 07 C 69/24, C 07 C 67/38**

(21) Anmeldenummer : **81105556.5**

(22) Anmeldetag : **15.07.81**

(54) Verfahren zur Herstellung von Alkylestern gesättigter aliphatischer Carbonsäuren.

(30) Priorität : **12.09.80 DE 3034421**

(43) Veröffentlichungstag der Anmeldung :
**24.03.82 Patentblatt 82/12**

(45) Bekanntmachung des Hinweises auf die Patenterteilung : **20.02.85 Patentblatt 85/08**

(84) Benannte Vertragsstaaten :
**AT BE FR GB IT NL**

(56) Entgegenhaltungen :
**FR-A- 1 402 383**
**US-A- 3 996 164**

(73) Patentinhaber : **CHEMISCHE WERKE HÜLS AG**
**Postfach 1320**
**D-4370 Marl 1 (DE)**

(72) Erfinder : **Müller, Wolfgang Hans Eduard, Dr.**
**Bitterfelder Strasse 9 a**
**D-4370 Marl (DE)**
Erfinder : **Hofmann, Peter, Dr.**
**Lipper Weg 193**
**D-4370 Marl (DE)**

Jouve, 18, rue St-Denis, 75001 Paris, France

**Beschreibung**

Es ist bekannt, daß man durch Umsetzung von Olefinen mit Kohlenmonoxid und einer H-aciden Komponente, wie z. B. Alkanolen in Gegenwart eines Katalysators, der ein Metall der 8. Nebengruppe des Periodischen Systems der Elemente und gegebenenfalls einen Promotor enthält, Fettsäureester herstellen kann (J. Falbe, Synthesen mit Kohlenmonoxid, Springer-Verlag, Berlin, Heidelberg, New York, 1967).

Als besonders bevorzugte Variante dieser als Hydrocarboxilierung bezeichneten Reaktion hat sich die Umsetzung in Gegenwart von kobalthaltigen Katalysatoren erwiesen. Eine besondere bevorzugte Ausführungsform besteht darin, daß man zusätzlich einen Promotor, und zwar insbesondere Pyridin oder ein nicht-orthosubstituiertes Alkylpyridin zusetzt.

Die Wirtschaftlichkeit eines solchen Verfahrens zur Herstellung von Fettsäureestern hängt in entscheidender Weise von der zu erzielenden Esterausbeute ab. Diese wird z. B. bei einer Verfahrensweise, bei der nicht umgesetzte Einsatz- und Hilfsstoffe in die Hydrocarboxilierungsstufe zurückgeführt werden, durch die Bildung von solchen Nebenprodukten beeinträchtigt, die höher als die gewünschten Reaktionsprodukte sieden (sogenannte Hochsieder). Durch die Bildung dieser Hochsieder kommt es nicht nur zu einem erhöhten Olefinverbrauch pro Gewichtseinheit des gewünschten Endproduktes, sondern beim notwendigen Abtrennen dieser Hochsieder auch zu Verlusten anderer wertvoller Einsatzstoffe.

Aufgabe der vorliegenden Erfindung war es daher, ein Verfahren zu entwickeln, bei dem der Anteil an Hochsiedern möglichst gering ist.

Diese Aufgabe wurde überraschenderweise durch die in den Ansprüchen beschriebenen Maßnahmen gelöst. Überraschenderweise deshalb, weil bei den Verfahren des relevanten Standes der Technik gerade der Zusatz von Wasser als kritische oder günstige Verfahrensmaßnahme dargestellt wird, um z. B. die Ausbeute an Fettsäureestern zu erhöhen (vgl. DE-OS 22 63 907, Seite 3, Absatz 2).

Das erfindungsgemäße Verfahren kann im Prinzip bei allen Hydrocarboxilierungsreaktionen, die in Gegenwart eines kobalthaltigen Katalysators durchgeführt werden, angewandt werden (z. B. Verfahren gemäß US-PS 3 507 891 und deutscher Patentanmeldung P 29 12 489.8). So ist vor allem die Wahl des eingesetzten Olefins unkritisch, d. h., es können sowohl geradkettige oder verzweigte α-Olefine als auch Olefine mit innenständiger Doppelbindung eingesetzt werden. Aber auch Olefine mit mehr als einer Doppelbindung und solche mit Substituenten, wie z. B. Aryl-, Cyano-, Carboximethyl- und Hydroxylgruppen, sind geeignet.

Im allgemeinen werden Olefine mit 2 bis 40, vorzugsweise mit 4 bis 20 Kohlenstoffatomen, eingesetzt, die nach bekannten Verfahren des Standes der Technik erhalten werden können. So können z. B. α-Olefine durch die Aufbaureaktion von Ethylen nach Ziegler oder durch Wachscracken, Olefine mit innenständiger Doppelbindung, die bevorzugt beim erfindungsgemäßen Verfahren eingesetzt werden, durch katalytische Dehydrierung von Paraffinen oder durch Chlorierung von Paraffinen und anschließender Dehydrochlorierung der Chlorparaffine gewonnen werden (GB-PS 1 037 868).

Bei dem zuletzt genannten Verfahren werden in der Regel Paraffinschnitte, d. h., Gemische unterschiedlicher C-Zahl eingesetzt, so daß auch die erhaltenen Olefine keine einheitliche C-Zahl aufweisen.

Außerdem kommen in diesen Olefin-Gemischen natürlich alle denkbaren isomeren Formen vor.

Neben den reinen — gegebenenfalls substituierten — Olefinen können auch solche mit einem Gehalt an Paraffinen, z. B. bis zu 85 Gewichtsprozent, eingesetzt werden. Der Paraffingehalt rührt daher, daß bei der Olefinherstellung keine vollständigen Umsätze erreicht und die nicht umgesetzten Paraffine nicht oder nicht vollständig abgetrennt werden.

Nicht nur das eingesetzte Olefin, sondern auch die Art des Alkanols, das mit dem Olefin und Kohlenmonoxid umgesetzt wird, ist für das erfindungsgemäße Verfahren unkritisch. Im allgemeinen werden Alkanole mit 1 bis 10, vorzugsweise 1 bis 4 Kohlenstoffatomen, eingesetzt. Typische Vertreter aus der Gruppe der primären Alkanole sind z. B. Methanol, Ethanol, Propanol-(1) und Butanol-(1).

Weiterhin ist es unwesentlich, welche Kobaltverbindung bei der Hydrocarboxilierung verwendet wird. Carbonyle des Kobalts, z. B. Dikobaltoctacarbonyl, sind ebenso geeignet wie carbonsaure Kobaltsalze, wie z. B. Kobaltacetat, Kobaltnaphthenat und Kobalt-2-ethylhexanoat, und Salze des Kobalts mit anorganischen Säuren, wie z. B. Kobaltnitrat und Kobaltsulfat. Vorzugsweise kommen solche carbonsauren Kobaltsalze zum Einsatz, deren Anionen dem Säurerest der bei der Hydrocarboxilierung gebildeten Fettsäureester entsprechen.

Als Promotoren kommen Pyridin und alle nichtorthosubstituierten Alkylpyridine, wie z. B. 3- und 4-Picolin, 3,4- und 3,5-Lutidin und 3- und 4-Ethylpyridin bzw. Mischungen dieser Stoffe infrage.

Schließlich sind die Reaktionsbedingungen, unter denen die Hydrocarboxilierung durchgeführt wird, für das erfindungsgemäße Verfahren nicht von Bedeutung. Im allgemeinen werden Hydrocarboxilierungsverfahren bei Temperaturen von 80 bis 300, vorzugsweise 150 bis 200 °C, und Kohlenmonoxiddrucken von 10 bis 800, vorzugsweise 100 bis 300 bar, durchgeführt. Die Konzentration des als Katalysator eingesetzten Kobalts liegt je nach Art des zur Umsetzung gelangenden Olefins in einem Bereich von 0,005 bis 0,2 Grammatom Kobalt pro Mol Olefin, die Menge des als Co-katalysator verwendeten Pyridins und/oder nicht-orthosubstituierten Alkylpyridins in einem Bereich von 3 bis 100, vorzugsweise 5 bis 50 Mol pro Grammatom Kobalt und die Menge des pro Mol Olefin eingesetzten Alkanols in einem Bereich von 1 bis 20, vorzugsweise 1 bis 10 Mol.

Verfahrenskritisch für das vorliegende Verfahren ist jedoch die Abtrennung des im Reaktionsgemisch enthaltenen Wassers aus zur Rückführung in die Hydrocarboxilierungsstufe vorgesehenen Stoffströmen auf Werte von weniger als 1 Gewichtsprozent, bezogen auf eingesetztes Olefin. Vorzugsweise beträgt der Wassergehalt weniger als 0,5 und besonders bevorzugt weniger als 0,1 Gewichtsprozent, bezogen auf eingesetztes Olefin (Frischolefin plus gegebenenfalls rückgeführtes Olefin).

Die Abtrennung des Wassers kann durch bekannte Verfahren, wie z. B. Destillation, Azeotropdestillation, Behandlung mit wasserentziehenden Adsorptionsmitteln, z. B. Molekularsieben, Natriumsulfat und Silicagel, oder Ausfrieren erfolgen.

Die beanspruchte Maßnahme kann sowohl bei diskontinuierlicher als auch bei kontinuierlicher Verfahrensweise angewendet werden. Besonders vorteilhaft ist jedoch die Anwendung bei einem kontinuierlichen Verfahren, bei dem ein oder mehrere Stoffströme (nicht umgesetztes Olefin, Alkanol und Kohlenmonoxid sowie wiedergewonnener Katalysator) rückgeführt werden.

Im allgemeinen führt man das erfindungsgemäße Verfahren so durch, daß man das Reaktionsgemisch zunächst z. B. durch Destillation in mehrere Fraktionen zerlegt. Die Hauptfraktionen sind in aller Regel Alkanol, Olefin, Fettsäureester und Cokatalysator. Als Sumpfprodukte verbleiben die Hochsieder sowie ein das Kobalt enthaltender Rückstand. Bei dieser Aufarbeitungsweise befindet sich das abzutrennende Wasser nahezu ausschließlich in den Destillatfraktionen, die normalerweise — natürlich mit Ausnahme der Fettsäureester, die z. B. zu Tensiden weiterverarbeitet werden, — in die Hydrocarboxilierungsstufe zurückgeführt und vorher z. B. mit Hilfe einer der bereits beschriebenen Methoden soweit als möglich bzw. nötig von Wasser befreit werden.

Das erfindungsgemäße Verfahren wird anhand der nachfolgenden Beispiele näher erläutert.

Beispiel 1

Ein Gemisch folgender Einsatzstoffe wird in den nachstehend aufgeführten Molverhältnissen

— 1 Mol n-Dodecen (Isomerengemisch mit einem n-Dodecen-(1)-Anteil $\leqslant$ 1 Gewichtsprozent; Reinheit 90 Gewichtsprozent; Wassergehalt 0,001 Gewichtsprozent)
— 2 Mol Methanol (Reinheit 90 Gewichtsprozent; Wassergehalt 0,018 Gewichtsprozent)
— 0,3 Mol $\gamma$-Picolin (Reinheit 98 Gewichtsprozent; Wassergehalt 0,095 Gewichtsprozent)
— 0,03 Grammatom Kobalt in Form eines 8 gewichtsprozentigen Kobalt enthaltenden Rückstandes, der bei der destillativen Aufarbeitung von Hydrocarboxilierungsgemischen anfällt (Wassergehalt < 0,000 1 Gewichtsprozent)

(Die in den Einsatzstoffen enthaltenen Verunreinigungen sind durch Destillation nicht abgetrennte Nebenprodukte der Hydrocarboxilierungsreaktion).

kontinuierlich in einen Rühr-Autoklaven eingepumpt und dort unter den folgenden Bedingungen zur Umsetzung gebracht (= Reaktionszyklus 1):

| | |
|---|---|
| Reaktionstemperatur: | 185 °C |
| CO-Warmdurck: | 180 bar |
| (CO enthält 1 Vol-% $H_2$) Verweilzeit: | 1,6 h |

Das Reaktionsgemisch wird nach einer bei 40 °C und 1 bar durchgeführten Behandlung mit Luft (50 l Luft/l Reaktionsgemisch) folgender kontinuierlichen Aufarbeitung unterworfen:

In einem Fallfilmverdampfer (FFV) wird das gesamte Reaktionsgemisch in eine das unumgesetzte Methanol enthaltende Destillatfraktion (Wassergehalt 1,45 Gewichtsprozent) zerlegt und einen methanolfreien Sumpf.

Der methanolfreie Sumpf des Fallfilmverdampfers wird destillativ in als Kopffraktionen anfallendes $\gamma$-Picolin (Wassergehalt 0,090 Gewichtsprozent), Olefin (Wassergehalt 0,001 Gewichtsprozent) und Tridecansäuremethylester sowie in als Sumpfprodukte erhaltene Hochsieder und kobalthaltigen Rückstand (Wassergehalt < 1 Gewichtsprozent) getrennt).

Von den zur Rückführung bestimmten Stoffen (Methanol, $\gamma$-Picolin, Olefin und kobalthaltiger Rückstand) wird nur das Methanol durch nochmalige Destillation von Wasser befreit: Man erhält als Kopfprodukt dieser Destillationsstufe Methanol mit einem Wassergehalt von 0,021 Gewichtsprozent. Der zurückgeführte kobalthaltige Rückstand wird vor seinem Wiedereinsatz einer Regeneration mit Synthesegas (50 Mol-% $H_2$, 50 Mol-% CO) bei 200 bar und 170 °C unterziehen. Die übrigen Stoffe bedürfen keiner weiteren Behandlung. Um jedoch eine Anreicherung der Verunreinigungen in den rückgeführten Stoffströmen zu vermeiden, werden 5 % des Methanols und 4 % des Olefins ausgeschleust. Nach Ergänzung der durch Reaktion verbrauchten Stoffe (im Falle von Methanol und Olefin) bzw. der durch Ausschleusung sowie durch Nebenreaktionen oder im Laufe der Aufarbeitung entstandenen Verluste (im

**0 047 834**

Falle von Methanol, Olefin, γ-Picolin und Kobaltkatalysator) durch wasserfreie Frischsubstanzen wird unter Einhaltung der anfangs genannten Mengenverhältnisse und Reaktionsbedingungen erneut die Hydrocarboxilierungsreaktion durchgeführt.

Der sich bei einer solchen Verfahrensweise ergebende Zusammenhang zwischen dem Wassergehalt der in die Reaktion rückgeführten Stoffströme (aus dem vorhergehenden Reaktionszyklus), dem Olefinumsatz, der Menge der als Nebenprodukt anfallenden Hochsieder sowie Esterselektivität ist für 30 aufeinander folgende Reaktionszyklen in Tabelle 1 dargestellt.

4

Tabelle 1

| Reakt. zyklus | Wassergehalte (Gew.-%) | | | | | $\sum$ Wassergehalte[1] (Gew.-%) | Olefinumsatz % | Hochsieder (Gew.-%)[2] | Esterselektivität (Mol-%) |
|---|---|---|---|---|---|---|---|---|---|
| | Rück-Methanol Dest. des FFV | Rück-Methanol nochmals destilliert | Rück-$\gamma$- Picolin | Rück- Olefin | Kobalt- Rückstand | | | | |
| 1 | 3) | 3) | 3) . | 3) | 3) | 3) | . 60 | 1,2 | 96,8 |
| 2 | 1,45 | 0,021 | 0,090 | 0,001 | < 0,0001 | 0,022 | 57 | 1,0 | 97,0 |
| 5 | 1,40 | 0,018 | 0,085 | 0,001 | < 0,0001 | 0,021 | 57 | 1,0 | 97,0 |
| 10 | 1,54 | 0,020 | 0,103 | 0,001 | < 0,0001 | 0,025 | 63 | 1,4 | 96,6 |
| 20 | 1,55 | 0,022 | 0,110 | 0,002 | < 0,0001 | 0,027 | 61 | 1,3 | 96,7 |
| 30 | 1,47 | 0,020 | 0,102 | 0,001 | < 0,0001 | 0,025 | 60 | 1,2 | 96,8 |

1) Summe der Wassergehalte im Rück-Methanol (nochmals destilliert), Rück-γ-Picolin, Rück-Olefin und Kobalt-Rückstand, bezogen auf Einsatzolefin (-Rück- und Frischolefin)

2) bezogen auf Tridecansäuremethylester

3) Wassergehalte der Einsatzstoffe für Reaktionszyklus 1, siehe Text des Beispiels

Beispiel 2

Beispiel 1 wird wiederholt mit der Ausnahme, daß das Methanol aus dem mit Luft behandelten Reaktionsgemisch nicht über einen Fallfilmverdampfer, sondern über eine mit Raschigringen gefüllte Kolonne mit 20 theoretischen Böden abgetrennt wird. Die Hauptmenge des im Reaktionsgemisch enthaltenen Wassers gelangt bei einer solchen Verfahrensweise nicht in die Methanol-, sondern in die Picolinfraktion und wird aus dieser durch Behandeln mit Molekularsieb entfernt.

Der sich bei einer solchen Verfahrensweise ergebende Zusammenhang zwischen dem Wassergehalt der in die Reaktion rückgeführten Stoffströme (aus dem vorhergehenden Reaktionszyklus), dem Olefinumsatz, der Menge der als Nebenprodukt anfallenden Hochsieder sowie der Esterselektivität ist für 10 aufeinanderfolgende Reaktionszyklen in Tabelle 2 dargestellt.

(Siehe Tabelle 2 Seite 7 f.)

Tabelle 2

| Reakt. zyklus | Wassergehalte (Gew.-%) | | | | | $\sum$ Wassergehalte[1] (Gew.-%) | Olefinumsatz % | Hochsieder (Gew.-%)[2] | Esterselektivität (Mol-%) |
| --- | --- | --- | --- | --- | --- | --- | --- | --- | --- |
| | Rück-Methanol | Rück-$\gamma$-Picolin Destillat | Rück-$\gamma$-Picolin nach Molsiebbehandl. | Rück-Olefin | Kobalt-Rückstand | | | | |
| 1 | 3) | 3) | 3) | 3) | 3) | 3) | 59 | 1,4 | 96,6 |
| 2 | 0,012 | 2,73 | 0,008 | 0,001 | < 0,0001 | 0,005 | 60 | 1,5 | 96,5 |
| 5 | 0,020 | 3,02 | 0,015 | 0,002 | 0,0002 | 0,009 | 62 | 1,3 | 96,6 |
| 10 | 0,014 | 2,70 | 0,010 | 0,001 | < 0,0001 | 0,006 | 60 | 1,4 | 96,8 |

1) Summe der Wassergehalte im Rück-Methanol, Rück-γ-Picolin (mit Molsieb behandelt), Rückolefin und Kobalt-Rückstand, bezogen auf Einsatzolefin (-Rück- und Frischolefin)

2) bezogen auf Tridecansäuremethylester

3) Wassergehalte der Einsatzstoffe für Reaktionszyklus 1, siehe Text des Beispiels

0 047 834

## Beispiel 3

Beispiel 1 wird wiederholt mit der Ausnahme, daß für den Reaktionszyklus 1 ein Olefin (Reinheit 90 Gewichtsprozent ; Wassergehalt 0,02 Gewichtsprozent) eingesetzt wird, dessen Olefinanteil zu 30 Gewichtsprozent aus einem wie in Beispiel 1 verwendeten Isomerengemisch von n-Dodecenen und zu 70 Gewichtsprozent aus n-Dodecen-(1) besteht und daß die Hydrocarboxilierungsreaktion bei einer Temperatur von 170 °C und einem Druck von 270 bar durchgeführt wird. Das durch Reaktion verbrauchte bzw. durch Ausschleusung oder im Laufe der Aufarbeitung verlorene Olefin wird jedoch wieder durch wasserfreies n-Dodecen-(1) ersetzt.

Der sich bei einer solchen Verfahrensweise ergebende Zusammenhang zwischen dem Wassergehalt der in die Reaktion rückgeführten Stoffströme (aus dem vorhergehenden Reaktionszyklus), dem Olefinumsatz, der Menge der als Nebenprodukt anfallenden Hochsieder sowie der Esterselektivität ist für 10 aufeinander folgende Reaktionszyklen in Tabelle 3 dargestellt.

(Siehe Tabelle 3 Seite 9 f.)

Tabelle 3

| Reakt. zyklus | Wassergehalte (Gew.-%) | | | | | ΣWassergehalte[1] (Gew.-%) | Olefinumsatz % | Hochsieder (Gew.-%)[2] | Esterselektivität (Mol-%) |
| --- | --- | --- | --- | --- | --- | --- | --- | --- | --- |
| | Rück-Methanol Dest. des FFV | Rück-Methanol nochmals destilliert | Rück-$\gamma$-Picolin | Rück-Olefin | Kobalt-Rückstand | | | | |
| 1 | 3) | 3) | 3) | 3) | 3) | 3) | 67 | 1,1 | 97,0 |
| 2 | 1,37 | 0,018 | 0,095 | 0,001 | < 0,0001 | 0,021 | 67 | 1,0 | 96,8 |
| 5 | 1,45 | 0,021 | 0,090 | 0,001 | < 0,0001 | 0,021 | 69 | 0,9 | 97,2 |
| 10 | 1,42 | 0,021 | 0,092 | 0,001 | < 0,0001 | 0,022 | 66 | 1,2 | 96,7 |

1) Summe der Wassergehalte im Rück-Methanol (nochmals destilliert), Rück-γ-Picolin, Rückolefin und Kobalt-Rückstand, bezogen auf Einsatzolefin (-Rückolefin und frisches n-Dodecen-(1))

2) bezogen auf Tridecansäuremethylester

3) Wassergehalte der Einsatzstoffe für Reaktionszyklus 1, siehe Text des Beispiels

0 047 834

Beispiel 4

Beispiel 1 wird wiederholt mit der Ausnahme, daß

1. Methanol durch gleiche Molmenge Ethanol (Reinheit 90 Gewichtsprozent ; Wassergehalt 0,01 Gewichtsprozent) ersetzt wird,

2. γ-Picolin durch die gleiche Molmenge 4-Ethylpyridin (Wassergehalt 0,06 Gewichtsprozent) ersetzt wird,

3. die ausgeschleuste Alkanolmenge auf 2 % des Rück-Ethanols reduziert wird,

4. die ausgeschleuste Olefinmenge auf 3 % des Rück-Olefins verringert wird und

5. Wasser durch Azeotropdestillation mit Benzol als Schleppmittel aus dem als Destillat des Fallfilmverdampfers anfallenden wasserhaltigen Ethanol abgetrennt wird.

Der sich aus einer solchen Verfahrensweise ergebende Zusammenhang zwischen dem Wassergehalt der in die Reaktion rückgeführten Stoffströme (aus dem vorhergehenden Reaktionszyklus), dem Olefinumsatz, der Menge der als Nebenprodukt anfallenden Hochsieder sowie der Esterselektivität ist für 10 aufeinander folgende Reaktionszyklen in Tabelle 4 dargestellt.

(Siehe Tabelle 4 Seite 11 f.)

Tabelle 4

| Reakt. zyklus | Wassergehalte (Gew.-%) | | | | | $\sum$ Wassergehalte[1] (Gew.-%) | Olefinumsatz % | Hochsieder (Gew.-%)[2] | Esterselektivität (Mol-%) |
| | Rück-Ethanol Dest. des FFV | Rück-Ethanol nach Azeotropdest. | Rück-4-Ethyl- pyridin | Rück-Olefin | Kobalt-Rückstand | | | | |
|---|---|---|---|---|---|---|---|---|---|
| 1 | 3) | 3) | 3) | 3) | 3) | 3) | 36 | 0,7 | 97,0 |
| 2 | 1,10 | 0,020 | 0,060 | 0,001 | < 0,0001 | 0,022 | 33 | 0,7 | 97,5 |
| 5 | 1,03 | 0,030 | 0,062 | 0,001 | < 0,0001 | 0,027 | 35 | 0,6 | 97,2 |
| 10 | 1,10 | 0,023 | 0,064 | 0,001 | < 0,0001 | 0,024 | 35 | 0,7 | 96,9 |

1) Summe der Wassergehalte im Rück-Ethanol (nach Azeotropdestillation), Rück-4-Ethylpyridin, Rückolefin und Kobalt-Rückstand, bezogen auf Einsatzolefin (-Rück- und Frischolefin)

2) bezogen auf Tridecansäuremethylester

3) Wassergehalte der Einsatzstoffe für Reaktionszyklus 1, siehe Text des Beispiels

**0 047 834**

Vergleichsbeispiel

Beispiel 1 wird wiederholt mit der Ausnahme, daß auf eine Abtrennung des Wassers aus dem als Destillat des Fallfilmverdampfers anfallenden wasserhaltigen Methanol verzichtet wird.

Der sich bei einer solchen Verfahrensweise ergebende Zusammenhang zwischen dem Wassergehalt der in die Reaktion rückgeführten Stoffströme (aus dem vorhergehenden Reaktionszyklus), dem Olefinumsatz, der Menge der als Nebenprodukt anfallenden Hochsieder sowie der Ersterselektivität ist für 11 aufeinander folgende Reaktionszyklen in Tabelle 5 dargestellt.

(Siehe Tabelle 5 Seite 13 f.)

Tabelle 5

| Reakt. zyklus | Wassergehalte (Gew.-%) | | | | ∑ Wassergehalte[1] (Gew.-%) | Olefinumsatz % | Hochsieder (Gew.-%)[2] | Esterselektivität (Mol-%) |
| | Rück-Methanol Dest. des FFV | Rück-$\gamma$-Picolin | Rück-Olefin | Kobalt-Rückstand | | | | |
|---|---|---|---|---|---|---|---|---|
| 1 | 3) | 3) | 3) | 3) | 3) | 60 | 1,1 | 97,0 |
| 2 | 1,48 | 0,102 | 0,001 | < 0,0001 | 0,43 | 58 | 2,5 | 95,2 |
| 3 | 2,3 | 0,18 | 0,001 | < 0,0001 | 0,67 | 60 | 3,2 | 94,7 |
| 5 | 3,7 | 0,30 | 0,001 | < 0,0001 | 1,08 | 62 | 4,3 | 93,5 |
| 7 | 4,9 | 0,33 | 0,001 | < 0,0001 | 1,42 | 61 | 4,6 | 93,0 |
| 9 | 6,0 | 0,47 | 0,001 | < 0,0001 | 1,75 | 61 | 5,0 | 92,2 |
| 11 | 6,6 | 0,49 | 0,001 | < 0,0001 | 1,91 | 59 | 4,8 | 92,1 |

1) Summe der Wassergehalte im Rückmethanol, Rück-γ-Picolin, Rück-Olefin und Kobalt-Rückstand, bezogen auf Einsatzolefin (-Rück- und Frischolefin)

2) bezogen auf Tridecansäuremethylester

3) Wassergehalte der Einsatzstoffe für Reaktionszyklus 1, siehe Text des Beispiels

**Ansprüche**

1. Verfahren zur Herstellung von Alkylestern gesättigter aliphatischer Carbonsäuren durch Umsetzung von Olefinen mit Kohlenmonoxid und Alkanol in Gegenwart eines Katalysators, bestehend aus einer Kobaltverbindung und Pyridin und/oder einem nicht-ortho-substituierten Alkylpyridin bei erhöhtem Druck und erhöhter Temperatur und Rückführung nicht umgesetzter Einsatzstoffe, dadurch gekennzeichnet, daß man das im Reaktionsgemisch enthaltene Wasser bei der Aufarbeitung des Reaktionsgemisches aus den für die Rückführung bestimmten Stoffströmen auf Werte von weniger als 1 Gewichtsprozent, bezogen auf eingesetztes Olefin, abtrennt.

2. Verfahren nach Anspruch 1, dadurch gekennzeichnet, daß man Olefine einsetzt, die durch katalytische Dehydrierung von Paraffinen oder durch Chlorierung von Paraffinen und anschließender Dehydrochlorierung der Chlorparaffine hergestellt werden.

3. Verfahren nach den Ansprüchen 1 und 2, dadurch gekennzeichnet, daß der Wassergehalt der rückgeführten Stoffströme weniger als 0,5 Gewichtsprozent, bezogen auf eingesetztes Olefin, beträgt.

4. Verfahren nach Anspruch 3, dadurch gekennzeichnet, daß der Wassergehalt der rückgeführten Stoffströme weniger als 0,1 Gewichtsprozent, bezogen auf eingesetztes Olefin, beträgt.

**Claims**

1. A process for the production of alkyl esters of saturated aliphatic carboxylic acids by reaction of olefins with carbon monoxide and alkanol at elevated pressure and elevated temperature in the presence of a catalyst composed of a cobalt compound and pyridine and/or a non-orthosubstituted alkylpyridine, with recycling of unconverted starting materials, characterised in that water present in the reaction mixture is separated from the recycled streams of material during the working up of the reaction mixture down to a concentration of less than 1 per cent by weight, based on olefin introduced.

2. A process according to claim 1, characterised in that olefins are used which have been produced by catalytic dehydrogenation of paraffins or by chlorination of paraffins and subsequent dehydrochlorination of the chloroparaffins.

3. A process according to claim 1 or 2, characterised in that the water content of the recycled streams amounts to less than 0.5 per cent by weight, based on olefin introduced.

4. A process according to claim 3, characterised in that the water content of the recycled streams amounts to less than 0.1 per cent by weight, based on olefin introduced.

**Revendications**

1. Procédé de préparation d'esters d'alkyle d'acides carboxyliques aliphatiques saturés, par réaction d'oléfines sur le monoxyde de carbone et un alcanol en présence d'un catalyseur formé d'un composé du cobalt et de pyridine et/ou d'une alkylpyridine non-substituée en ortho, à pression élevée et à température élevée et avec recyclage des matières premières non converties, caractérisé par le fait que, lors du traitement du mélange réactionnel, on sépare l'eau contenue dans le mélange réactionnel des courants de matière destinés au recyclage, jusqu'à des teneurs de moins de 1 % en poids, relativement à l'oléfine mise en œuvre.

2. Procédé selon la revendication 1, caractérisé par le fait que l'on utilise des oléfines que l'on prépare par déshydrogénation catalytique de paraffines ou par chloration de paraffines et par déchlorydratation subséquente des chloroparaffines.

3. Procédé selon les revendications 1 et 2, caractérisé par le fait que la teneur en eau des courants de matière recyclés est de moins de 0,5 % en poids, relativement à l'oléfine mise en œuvre.

4. Procédé selon la revendication 3, caractérisé par le fait que la teneur en eau des courants de matière recyclés est de moins de 0,1 % en poids, relativement à l'oléfine mise en œuvre.